# EUROPEAN PATENT APPLICATION

(11) **EP 1 466 595 A1**
(43) Date of publication of application: **13.10.2004**
(21) Application number: 04077077.8
(22) Date of filing: 03.11.2000
(51) Int. Cl.: A61K 9/14, A61K 9/16, A61K 9/00

(54) **Apparatus and process for preparing crystalline particles**

(30) Priority: 03.11.1999 GB 9925934; 06.10.2000 GB 0024511
(62) Divisional of application: 00973049.0
(71) Applicant: GLAXO GROUP LIMITED, Greenford, Middlesex UB6 ONN (GB)
(72) Inventor: Ferrie, Alan Ronald, c/o GlaxoSmithKline, Ware, Hertfordshire SG12 0DP (GB); Savage, Andrew Patrick, c/o GlaxoSmithKline, Ware, Hertfordshire SG12 0DP (GB)
(74) Representative: Shore, Andrew David

(57) **Abstract**

An apparatus for preparing crystalline particles of a substance suitable for inhalation therapy comprises
(i) a first reservoir 3 of said substance dissolved in a liquid solvent;
(ii) a second reservoir 3 of liquid antisolvent for said substance which is miscible with the liquid solvent;
(iii) a cylindrical mixing chamber 2 having first and second tangential inlet ports and an axial outlet port;
(iv) means for delivering the contents of the first and second reservoirs to the mixing chamber in a stream via the first and second inlet ports respectively at independent controlled flow rate, the streams of liquid from t he i nlet a nd o utlet ports being intimately mixed in the cylindrical mixing chamber through formation of a vortex thereby causing precipitation of crystalline particles of the substance; and
(vi) means 5 for collecting crystalline particles suspended in the stream of liquid discharged from the outlet port of the mixing chamber.

## Description

This invention relates to a novel apparatus for preparing crystalline particles suitable for inhalation therapy. There is also provided a process for preparing the same.

Industrial processes for production of many products, particularly pharmaceutical products, require the preparation of pure substances of a defined particle size distribution. Pure substances are frequently prepared by precipitation from solutions of lesser purity. When precipitation takes place relatively slowly (eg over a matter of hours), crystals are grown which are frequently of an non-uniform shape and relatively large size.

In the field of inhalation therapy, therapeutic molecules are generally desired of a particle size "suitable for inhalation", which is a term generally taken to indicate an aerodynamic diameter between 1 and 10 µm, especially 1 and 5 µm, particularly 1 and 3 µm. Carrier molecules (such as lactose) for inhaled therapeutic preparations are typically desired of a significantly larger aerodynamic diameter so that they do not penetrate into the upper respiratory tract to the same degree as the active ingredient and an aerodynamic diameter of 100 to 150 µm is generally considered suitable. However this is a generalisation and for some purposes it may well be preferred to use a lower particle size for the carrier, even one comparable to that of the therapeutic substance.

Particles of the desired particle size for inhalation therapy are conventionally prepared by milling or micronisation. These processes, depending on the precise conditions adopted, are capable of generating particle distributions which include fractions having particles with the appropriate size. Milling is suitable for preparing particles of the larger size indicated above and micronisation of the smaller size indicated above. However, there are a number of disadvantages associated with milling and micronisation processes including that the fraction having the desired particle size may be relatively small, that there may be generated a significant fraction of particles that are finer than is desired (which may be deleterious eg if it affects bioavailability) and that product losses generally may be considerable. A further property of micronised products is that the surfaces of the particles generated are generally substantially amorphous (i.e. have minimal crystallinity). This may be undesirable when there exists a tendency for the amorphous regions to convert to a more stable crystalline state. Additionally, amorphous substances may be more susceptible to undesirable moisture uptake than crystalline substances. Furthermore the effectiveness of a micronisation process is highly sensitive to the hardness of the crystals of substance to be micronised with the result that some substances are difficult to reduce below a certain size. In these circumstances further micronisation tends to result in broadening of the particles size distribution (largely due to generation of more hyperfine particles) rather than reduction of the mass median diameter (MMD).

Rapid precipitation (eg by dilution of a solution with an anti-solvent) may give rise to crystalline particles which could be of suitable size, however this technique is notoriously difficult to control and has not found widespread acceptance in the pharmaceutical industry, particularly in relation to inhalation products.

Methods of mixing liquids using a "vortex mixer" are described inter alia in EP-A-646407, EP-A-0449454, GB2253162B and EP-B-0153843.

We have now invented a novel process and apparatus for preparing particles suitable for inhalation therapy which overcomes or substantially mitigates one or more of the above mentioned disadvantages.

Thus according to a first aspect of the invention there is provided a process for preparing crystalline particles of a substance suitable for inhalation therapy which comprises (i) admitting a stream of solution of the substance in a liquid solvent and a stream of liquid antisolvent for said substance which is miscible with the liquid solvent tangentially into a cylindrical mixing chamber having an axial outlet port such that said streams are thereby intimately mixed through formation of a vortex and precipitation of crystalline particles of the substance is thereby caused; and (ii) collecting the resultant crystalline particles suspended in the stream of liquid discharged from the outlet port of the mixing chamber.

A particular advantage of the process is that is capable of running continuously (subject to adequate supply of solution and anti-solvent) even if, for a particular application, it may be desired to run it only for a relatively short time.

Further advantages include the fact that "tight" distributions of particles (i.e. distributions with high uniformity index) may be obtained centered around different MMD's, as desired, through simple modification of the operating parameters. This gives the process according to the invention a flexibility not possessed by conventional micronisation processes for the reasons given above.

Preferably, prior to collecting the resultant crystalline particles, the stream of liquid discharged from the outlet port of the mixing chamber is diluted with liquid anti-solvent.

According to a second aspect of the invention there is provided an apparatus for preparing crystalline particles of a substance suitable for inhalation therapy which comprises
(i) a first reservoir of said substance dissolved in a liquid solvent;
(ii) a second reservoir of liquid antisolvent for said substance which is miscible with the liquid solvent;
(iii) a cylindrical mixing chamber having first and second tangential inlet ports and an axial outlet port;
(iv) means for delivering the contents of the first and second reservoirs to the mixing chamber in a stream via the first and second inlet ports respectively at independent controlled flow rate wherein the streams of liquid from the inlet and outlet ports are intimately mixed in the cylindrical mixing chamber through formation of a vortex and precipitation of crystalline particles of the substance is thereby caused; and
(vi) means for collecting crystalline particles suspended in the stream of liquid discharged from the outlet port of the mixing chamber.

Desirably the means for delivering the contents of the first and second reservoirs to the mixing chamber via the first and second inlet ports respectively at independent controlled flow rate comprises one or more pumps. Preferably a pump will be provided for each of the first and second reservoirs. A range of pumps are available and may be suitable for the apparatus according to the invention. The pump may, for example, be a peristaltic pump or a gear pump. Pumps which are essentially non-pulsing are preferred. When a peristaltic pump is used it may be preferred to use it in conjunction with a pulse dampener.

The contents of the first and second reservoirs may be delivered to the mixing chamber at a range of flow rates which will be selected and optimised according to the nature of the substance, the solvent and the antisolvent. Typically flow rates will be in the range of 5 to 100 I/hr.

The mixing chamber may be manufactured from a range of conventional materials: however these will preferably be selected so as to be unreactive with the substance, the solvent or the anti-solvent. The mixing chamber may be of any suitable size, whether of a size suitable for bench-scale preparation, industrial pilot scale preparation or industrial manufacturing scale.

Particles suspended in the liquid discharged from the outlet port of the mixing chamber may be collected by means of one of a number of conventional particle capturing techniques. For example, filtration means may be used; a wide range of suitable filters are known to persons skilled in the art. The filter may be provided with a drying facility. Filtration will preferably be under pressure; pressures of around 3 bar (gauge) are suitable.

In case caking of the filtered particles should occur, the particles may be redispersed by dispersion in a non-solvent (eg isooctane for many applications) and subjected to sonication, followed by subsequent drying (eg by evaporation) or spray-drying.

A filter cake recovered from a pressure filter for formulation into either dry powder or metered dose inhalers may be sieved.

In a system where the crystallisation of the substance out of solution is essentially complete, the outflow from the mixing chamber may be fed to a spray-drying facility directly such that the solvent/antisolvent mixture is vaporised and the particles collected dry.

Another method of collection is by use of a hydrocyclone for liquid removal. The resultant slurry may be spray dried or dried by other conventional means. A particular advantage of use of a hydrocyclone is that it can be used to further refine the resultant particle distribution by removal of a proportion from either the course end or the fine end of the distribution through appropriate choice of the operating parameters.

In a further method of collection, the suspension of particles is subjected to ultrafiltration to remove liquid. The resultant slurry may be spray dried or dried by other conventional means.

The apparatus of the present invention may further comprise means for diluting the stream of liquid discharged from the outlet port of the mixing chamber by delivering a stream of anti-solvent to said stream of liquid downstream of the outlet port of the mixing chamber and upstream of the means for collecting the crystalline particles.

The diluting means may comprise a mixing or quenching head or vessel having an inlet for the stream of liquid discharged from the outlet port of the mixing chamber, another inlet for the stream of anti-solvent and an outlet for the resulting mixture.

The dilution of the stream of liquid discharged from the outlet port of the mixing chamber with anti-solvent seeks to minimise unwanted ripening of the crystalline particles: it allows further dilution of the solvent to reduce crystal growth, particularly when producing solvent-rich suspensions. Moreover, it allows the crystalline particles to be washed to assist removal of residual solvent, particularly when the particles are collected on a filter. This dilution also assists in reducing caking and agglomeration.

As a further aspect of the invention we provide a process for preparing crystalline particles of a substance suitable for inhalation therapy using an apparatus according to the invention which comprises
(i) delivering the contents of the first and second reservoirs to the mixing chamber via the first and second inlet ports respectively at independent controlled flow rate; and
(iii) collecting the crystalline particles suspended in the stream of liquid discharged from the outlet port of the mixing chamber.

The process is suitable for preparing particles of substances which are pharmaceutical or carrier substances suitable for inhalation therapy. The preferred process wherein the benefits of the invention are greatest is a process for preparing pharmaceutical substances suitable for inhalation therapy, especially a process for preparing pharmaceutical substances suitable for inhalation therapy having a particle size (eg an aerodynamic diameter) between 1 and 10 µm.

Examples of pharmaceutical substances suitable for inhalation therapy include analgesics, e.g., codeine, dihydromorphine, ergotamine, fentanyl or morphine; anginal preparations, e.g., diltiazem; antiallergics, e.g., cromoglycate, ketotifen or nedocromil; antiinfectives e.g., cephalosporins, penicillins, streptomycin, sulphonamides, tetracyclines and pentamidine; antihistamines, e.g., methapyrilene; anti-inflammatories, e.g., beclomethasone, fluticasone, flunisolide, budesonide, rofleponide, mometasone or triamcinolone; antitussives, e.g., noscapine; bronchodilators, e.g., albuterol, salmeterol, ephedrine, adrenaline, fenoterol, formoterol, isoprenaline, metaproterenol, phenylephrine, phenylpropanolamine, pirbuterol, reproterol, rimiterol, terbutaline, isoetharine, tulobuterol, (-)-4-amino-3,5-dichloro-α-[[[6-[2-(2-pyridinyl)ethoxy]hexyl]methyl] benzenemethanol or 4-hydroxy-7-[2-[[2-[[3-(2-phenylethoxy)propyl]sulfonyl]ethyl]amino]ethyl-2(3H)-benzothiazolone; diuretics, e.g., amiloride; anticholinergics, e.g., ipratropium, tiotropium, atropine or oxitropium; hormones, e.g., cortisone, hydrocortisone or prednisolone; xanthines, e.g., aminophylline, choline theophyllinate, lysine theophyllinate or theophylline; therapeutic proteins and peptides, e.g., insulin or glucagon; and salts, esters and solvates of any of the above.

Pharmaceutical substances of particular interest include fluticasone, beclomethasone, salmeterol, salbutamol or an ester, salt or solvate thereof. The substance of most interest is fluticasone propionate. Salmeterol xinafoate is also of particular interest. Fluticasone propionate and salmeterol xinafoate are of particular interest when used therapeutically in combination.

Examples of carrier substances include lactose.

The solvent and antisolvent liquids will be selected so as to be appropriate for the substance. It is also necessary that they are readily miscible in the proportions employed. Suitable combinations of solvent/antisolvent include acetone/water, ethanol/isopropanol, methanol/isopropanol, methanol/water, dimethylformamide/water, dimethylacetamide/water, dimethylsulphoxide/water, acetone/isooctane and reciprocal pairs.

1,1,1,2-tetrafluoroethane (HFA134a) and 1,1,1,2,3,3,3-heptafluoro-n-propane (HFA227) are also potential solvents or antisolvents which may be paired eg with ethanol. However the use of these gases in liquefied form would require the use of cold or pressurised equipment.

For generation of small particles by the process according to the invention, it is preferred that the difference between the dissolution properties of the solvent and anti-solvent be as great as possible. For reasons of industrial efficiency (particularly in order to reduce the throughput volumes of liquid) it is preferred to use concentrations of substance in solvent which are as high as possible. Nevertheless the solutions must be stable and not prone to crystallisation before discharge into the mixing chamber. With this end in mind, it may be preferred to use the solution of the substance in the solvent at elevated temperature. It may also be preferable to cool the anti-solvent.

In order to prevent premature precipitation of the dissolved substance in the lines it will generally be desired to prime the apparatus by first pumping it with solvent. It may be preferred to prime the apparatus by pumping it with heated solvent, particularly when the dissolved substance is close to its solubility limit.

The optimum relative flow rates of substance/solvent solution and anti-solvent will generally depend on the solubility of the substance in the solvent relative to the anti-solvent. The lower this ratio is, the lower may be the flow rate of anti-solvent relative to the substance/solvent solution.

Higher flow rates of anti-solvent have a tendency to result in crystalline particles of smaller mean size.

When the substance is fluticasone propionate we prefer the solvent to be acetone and the anti-solvent to be water. Alternative preferred conditions are where the the solvent is acetone and the anti-solvent is isooctane.

When the substance is salmeterol xinafoate we prefer the solvent to be methanol or acetone (more preferably methanol) and the anti-solvent to be water or IMS (more preferably water). Alternative preferred conditions are where the the solvent is methanol and the anti-solvent is isooctane.

When the substance is salbutamol sulphate, we prefer the solvent to be water and the anti-solvent to be IMS.

When the substance is beclomethasone dipropionate we prefer the solvent to be IMS and the anti-solvent to be water.

When the substance is lactose we prefer the solvent to be water and the anti-solvent to be ethanol.

When the substance is budesonide, we prefer the solvent to be IMS and the anti-solvent to be water.

When the substance is formoterol fumarate or terbutaline sulphate we prefer the solvent to be methanol or acetone and the anti-solvent to be water or IMS.

Typical concentrations for solution of substance are 30-50 g/l although concentrations outside this range are not excluded. Indeed higher concentrations may well be preferred if attainable. Preferred flow rates for solution and anti-solvent are typically in the range 10-30 I/hr and 50-90 I/hr respectively. Example ranges of linear velocities of the solvent and anti-solvent streams as they enter the cylindrical mixing chamber are 2.8 to 3.5 m/s for the solvent stream and 4.9 to 6.3 m/s for the anti-solvent stream.

As a further aspect of the invention we provide a population of particles obtainable by a process according to the invention.

Particles of pharmaceutical or carrier substances may be obtained which are suitable for use in a pharmaceutical composition for inhalation therapy, such as dry powder composition (whether containing pure drug, or drug mixed with a carrier such as lactose) or a pressurised liquid aerosol formulation (eg a formulation comprising a hydrofluoroalkane propellant such as HFA134a or HFA227 or a mixture thereof).

A preferred inhalation device for dry powder compositions is the Diskus inhaler (described in US patents 5590645 and 5860419) which may be charged with "blister packs" containing the drug composition as described in US patent 5873360. An alternative inhalation device is the Turbohaler inhaler as described in Canadian patent 1178151.

Formulations of salmeterol xinafoate and fluticasone propionate blended with lactose which are suitable for blister packs in a Diskus inhaler typically contain: 72.5µg salmeterol xinafoate to 12.5mg lactose and 50, 100, 250 or 500µg fluticasone propionate to 12.5mg lactose respectively.

Formulations of salmeterol xinafoate and fluticasone propionate combination blended with lactose which are suitable for blister packs in a Diskus inhaler typically contain: 72.5µg salmeterol xinafoate and 50, 100, 250 or 500µg fluticasone propionate to 12.5mg lactose.

Formulations of salbutamol sulphate and beclomethasone dipropionate blended with lactose which are suitable for blister packs in a Diskus inhaler typically contain: 200µg salbutamol sulphate to 12.5mg lactose and 50, 100, 200 or 400 µg beclomethasone propionate to 12.5mg lactose respectively.

Pressurised liquid formulations suitable for metered-dose inhalers will be retained in canisters, typically aluminium canisters (which may be plastics lined) which are provided with a metering valve of appropriate metering volume.

Formulations of fluticasone propionate in hydrofluoroalkane propellant will typically deliver 25, 50, 125 or 250 µg drug per actuation. Preferred formulations of fluticasone propionate in hydrofluoroalkane propellant consist of fluticasone propionate and HFA134a and are excipient free. Suitable concentrations of fluticasone propionate in HFA134a are around 0.04%, 0.08%, 0.16% and 0.33% w/w.

Formulations of salmeterol xinafoate in hydrofluoroalkane propellant will typically deliver 25 ug drug per actuation. Preferred formulations of salmeterol xinafoate in hydrofluoroalkane propellant consist of salmeterol xinafoate and HFA134a and are excipient free. A suitable concentration of salmeterol xinafoate in HFA134a is around 0.05% w/w.

Formulations of fluticasone propionate and salmeterol xinafoate combination in hydrofluoroalkane propellant will typically deliver 50, 125 or 250 µg fluticasone propionate and 25 µg salmeterol xinafoate per actuation. Preferred formulations of fluticasone propionate and salmeterol xinafoate combination in hydrofluoroalkane propellant consist of fluticasone propionate and salmeterol xinafoate and HFA134a and are excipient free. Suitable formulations in HFA134a contain salmeterol xinafoate and fluticasone propionate in approximate concentrations (%) as follows: 0.05/0.07; 0.05/0.17; 0.05/0.33.

Formulations of salbutamol sulphate in hydrofluoroalkane propellant will typically deliver 100 µg drug per actuation. Preferred formulations of salbutamol sulphate in hydrofluoroalkane propellant consist of salbutamol sulphate and HFA134a and are excipient free. A suitable concentration of salbutamol sulphate in HFA134a is around 0.16% w/w.

We also provide a pharmaceutical composition comprising a population of particles prepared according to the invention.

The advantages that the invention may possess include the fact that the process may be performed in a continuous manner without requirements for batch processing, that process may be scaled up with relative ease and that the apparatus and process are capable of producing particle size distributions of very high uniformity index.

Embodiments of the present invention will now be described, by way of example only, with reference to the accompanying diagrammatic drawings, in which:
Figure 1 is one embodiment of apparatus for carrying out the invention;
Figure 2 is a graph of particle size against %volume;
Figures 3 and 4 are bar charts of fine particle mass;
Figure 5 is another embodiment of apparatus for carrying out the invention;
Figure 6 is a cross-sectional planar view of a vortex mixing head; and
Figures 7 and 8 are graphs of time after collection against particle size.

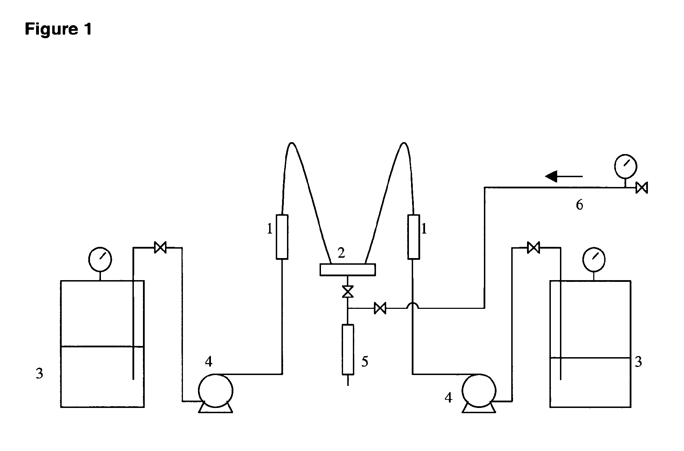

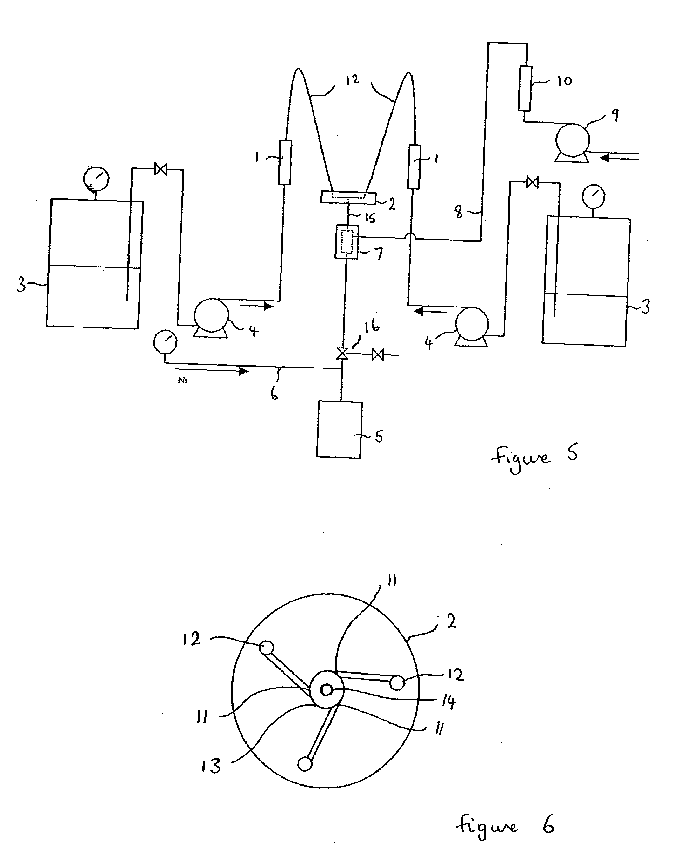

Referring to figures 1 and 5, flow meters 1 control the flow of solution and anti-solvent from pressure vessel reservoirs 3 to the vortex mixing head 2 (shown in side view) by means of pumps 4. The streams of solution and anti-solvent enter the vortex mixing head via conduits 12. Crystals of product are collected by filtration at pressure filter 5 supplied with nitrogen gas (regulated to <=4 bar) through lines 6.

The apparatus of figure 5 is preferred to the apparatus of figure 1. The apparatus of figure 5 additionally comprises a quenching head 7 located downstream of vortex mixing head 2 and upstream of pressure filter 5. A conduit 15 links the outlet port of the vortex mixing head with an inlet port of the quenching head. The quenching head is supplied with anti-solvent through line 8 via pump 9 and flow meter 10 from a reservoir (not shown). Pump 9 may be the same type of pump as pumps 4. In the following examples they are all gear pumps.

A bypass valve 16 is located at the outflow of the quenching head. This allows the slurry produced at the start of a run, during which target flow conditions have not been met, to be collected in a separate vessel to that in which the product obtained at optimum conditions is collected.

The addition of a quenching head for diluting the stream of liquid discharged from the outlet port of the vortex mixing head with anti-solvent is intended to reduce particle growth and thus ripening of the particle size distribution following precipitation within the vortex mixing head. Particle growth and ripening of the particle size distribution are particularly a problem when the stream of liquid discharged from the outlet port of the vortex mixing head is solvent-rich.

To achieve temperature control within the system, cooling coils (not shown) are incorporated in the anti-solvent reservoirs; these coils form a circuit connected to a refrigerated circulating bath (not shown). Also, the solution reservoir is contained within a heating bath (not shown).

With reference to figure 6, a vortex mixing head 2 has three tangential inlet ports 11, although, in the following examples, only two of these are utilised. One tangential inlet port is supplied with a stream of solution via conduit 12. Another tangential inlet port is supplied with a stream of anti-solvent via another conduit 12. The vortex mixing head comprises a cylindrical vortex chamber 13 in communication with the tangential inlet ports which are in the circumferential wall of the chamber. This chamber is also in communication with an axial outlet port 14 located in an end wall of the chamber. The streams enter tangentially to swirl through the chamber to emerge at the outlet port, and in so doing, thorough mixing of the streams takes place.

In another embodiment (not shown), the vortex mixing head has a pair of diametrically opposed tangentially directed inlet ports.

Examples 1 to 5 use the apparatus of figure 1 whereas example 6 uses the apparatus of figure 5.

### Example 1

### Distributions of particles of crystalline fluticasone propionate

### Method of crystallisation

Water was pumped through a vortex mixing head at a flow rate of 90 I/hr using a gear pump (200.350 micropump, Micropump Inc). A solution of fluticasone propionate in acetone (concentration: 50g/l) was then pumped using a gear pump at a flow rate of 10 I/hr. Once the flows of both streams had stabilised, the eluted suspension was collected (approx. 300ml) and crystals isolated as quickly as practicable by pressure filtration at 3 bar (gauge) pressure. The filter cake produced was dry, so further drying by vacuum oven was not necessary.

Experiments were performed using vortex mixing heads of 4 different geometries.

### Particle size analysis

Crystals of fluticasone propionate produced by the above method were dispersed in a solution of lecithin (0.05% w/w) in 2,2,4-trimethylpentane (iso-octane) by sonication for up to 6 minutes. Sizing was then performed by laser diffraction (Malvern Mastersizer). Results are shown in Table 1. Particle distributions of suitable MMD and high uniformity index were obtained.

### Variation of operating parameters

Further experiments were performed to determine the effect of solution concentration and solution and anti-solvent flow-rate using vortex mixing head 2. Results are shown in Table 2.

### Comparison of distributions obtained with micronised sample

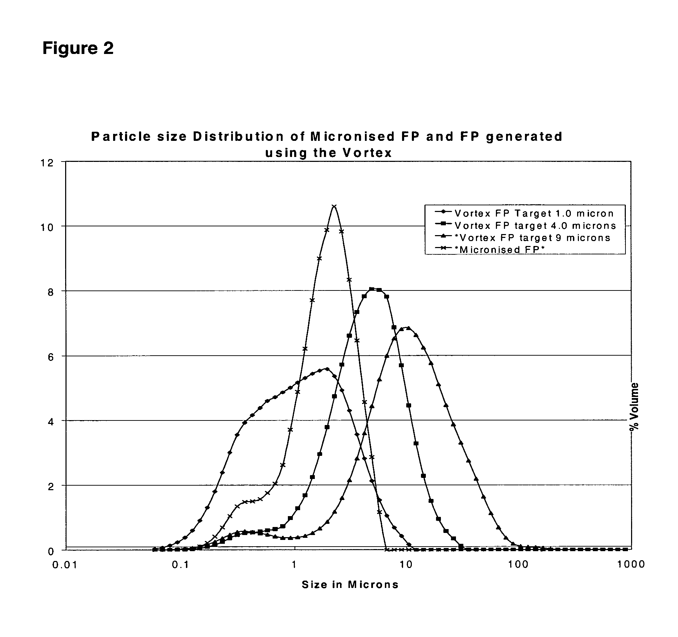

The particle size distributions (from Malvern Mastersizer) obtained for three experiments with fluticasone propionate where the target d₅₀ was 1, 4 and 9 microns were compared with a conventional micronised sample and the distributions shown in Figure 2. The operating parameters for these three experiments are shown in the last three rows of Table 2.

### Example 2

### Distributions of particles of crystalline salmeterol xinafoate

Particles of crystalline salmeterol xinafoate were generated using the method of Example 1 using conditions as follows:
Vortex mixing head: 3
Solution of salmeterol xinafoate in methanol: concentration 40g/l and 6 g/l
Solution flow rate: 10 l/hr
Water anti-solvent flow rate: 90 l/hr

Particle size analysis was performed as per Example 1 and results are shown in Table 3.

In Tables 1,2 and 3: d₅₀ = MMD and uniformity index (UI) = 100 x D₁₀/D₉₀

### Example 3

An aluminium canister may be charged with particles of (a) fluticasone propionate or (b) salmeterol xinafoate or (c) fluticasone propionate and salmeterol xinafoate prepared according to the invention. A metering valve (Valois) (63 µl metering volume) may be crimped on and liquefied HFA134a added through the valve.

Suitable canister formulation composition would be:
For a 250µg per dose product:: 40mg fluticasone propionate made up to 12g with HFA134a
For a 125µg per dose product: 20mg fluticasone propionate made up to 12g with HFA134a

### Example 4

A dry powder composition for inhalation from blister packs using a Diskus inhaler was prepared as follows:
Salmeterol xinafoate nominal d₅₀ 4.1 µm (prepared as per Table 3 row 2 with 4 minute sonication of filter cake dispersed in iso-octane followed by air drying): 72.5µg
Milled lactose (9.5% by weight less than 15 micron): 12.5mg

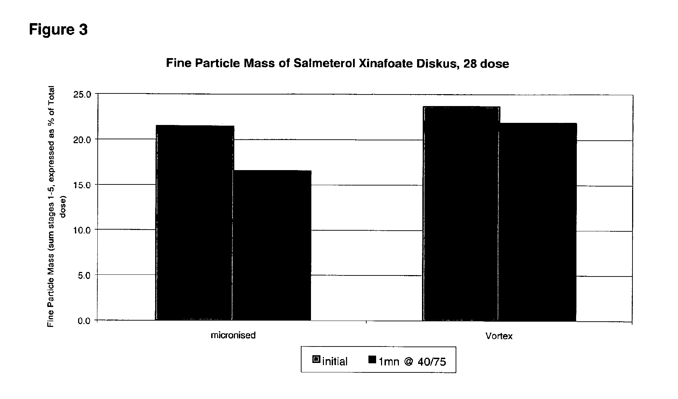

Blister packs were prepared and the fine particle mass (FPM) when delivered from Diskus inhaler (flow rate 60 I/min) as measured by a cascade impactor (defined as the fraction trapped in stages 1-5 of the cascade impactor; approximately equates to the fraction between 1-6 µm) was measured. Results, and comparison with salmeterol xinafoate prepared by conventional micronisation, are shown in Figure 3. Figure 3 also shows the FPM after storage for 1 month at 40°C and 75% humidity.

The batch prepared from salmeterol xinafoate prepared according to the method of the invention has very good FPM and storage stability relative to the batch prepared from salmeterol xinafoate prepared by the conventional micronisation technique.

### Example 5

A dry powder composition for inhalation from blister packs using a Diskus inhaler was prepared as follows:
Fluticasone propionate nominal d₅₀ 3.9 µm (prepared as per Table 2 row 2 with 4 minute sonication of filter cake dispersed in isopentane followed by air drying): 50µg
Milled lactose (9.5% by weight less than 15 micron): 12.5mg

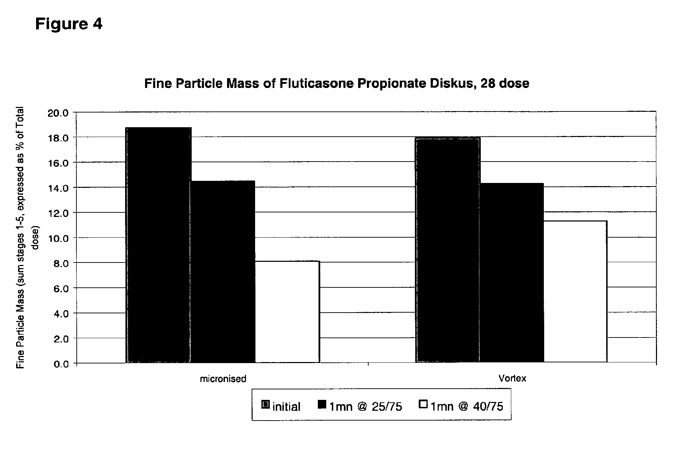

Blister packs were prepared and the FPM when delivered from Diskus inhaler (flow rate 60 I/min) as measured by a cascade was measured. Results, and comparison with fluticasone propionate prepared by conventional micronisation, are shown in Figure 4. Figure 4 also shows the FPM after storage for 1 month at 25°C and 75% humidity or storage at 40°C and 75% humidity.

The batch prepared from fluticasone propionate prepared according to the method of the invention has very good FPM and storage stability relative to the batch prepared from fluticasone propionate prepared by the conventional micronisation technique.

### Example 6

### Distributions of particles of crystalline fluticasone propionate

### Method of crystallisation

Water was pumped through vortex mixing head 1. A solution of fluticasone propionate in acetone was then pumped through the vortex mixing head. Once the flows of both streams had stabilised, the eluted suspension was quenched in the quenching head using a quench water flow of 90l/h and collected. The crystals were isolated as quickly as practicable by pressure filtration.

A variety of operating parameters were used to determine the effect of solution concentration and solution and anti-solvent flow-rates. Results are shown in Table 4.

### Particle size analysis

Crystals of fluticasone propionate produced by the above method were dispersed in a solution of lecithin (0.05% w/w) in 2,2,4-trimethylpentane (iso-octane) by sonication for 2 minutes. Sizing was then performed by laser diffraction (Malvern Mastersizer S). Results are shown in Table 4. Particle distributions of suitable MMD were generally obtained.

### Suspension stability

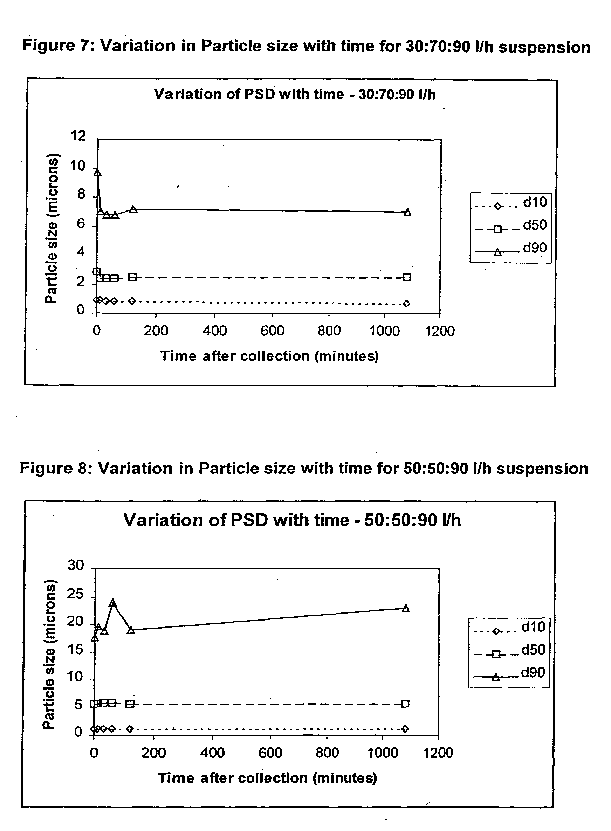

The effectiveness of the quenching head was evaluated by monitoring the change in particle size distribution over time for a suspension collected at the outflow of the head. Solutions were produced at two flow conditions, namely 30:70:90 and 50:50:90 I/h (solution:antisolvent:quench flow), and evaluated over a period of 18 hours at ambient conditions. D10, d50 and d90 values at measured timepoints are given in figures 7 and 8. Particle growth can be seen to be minimal in both cases.

### Sieving of product for inhalation

Aliquots of filter cake recovered from the pressure filter after a 30:70:90 I/h (solution:antisolvent:quench flow) run were sieved, analysed by laser diffractometry and compared to an unsieved fraction from the same filter cake. Results are shown in Table 5. Sieving appears to have no effect on the particle size distribution of the recovered product but improves the characteristics of the filter cake thereby rendering the product more suitable for formulation into either dry powder or metered dose inhalers. To save time in a commercial process, the 335 micron aperture screen is preferred.

**Table 1:**

| **Evaluation of Vortex Mixer Heads for Fluticasone Propionate** | | | | |
|---|---|---|---|---|
| Head number | d₅₀ (µm) | d₁₀(µm) | d₉₀(µm) | UI |
| 1 | 2.49 | 0.46 | 9.27 | 5.0 |
| 2 | 1.50 | 0.36 | 4.91 | 7.3 |
| 3 | 2.63 | 0.53 | 8.09 | 6.6 |
| 4 | 3.34 | 0.54 | 12.65 | 4.3 |

**Table 2:**

| **Evaluation of Operating Parameters for Fluticasone Propionate** | | | | | | |
|---|---|---|---|---|---|---|
| Solution concentration (gl⁻¹) | Solution flow rate (Ih⁻¹) | Anti-solvent flow rate (Ih⁻¹) | d₅₀ (µm) | d₁₀ (µm) | d₉₀ (µm) | UI |
| 40.0 | 10 | 50 | 2.3 | 0.6 | 6.5 | 9.2 |
| 40.0 | 30 | 70 | 3.9 | 1.0 | 12.9 | 7.8 |
| 40.0 | 50 | 90 | 6.0 | 1.6 | 13.8 | 11.6 |
| 47.5 | 30 | 50 | 6.3 | 2.2 | 13.5 | 16.3 |
| 47.5 | 30 | 70 | 4.9 | 1.5 | 11.3 | 13.3 |
| 47.5 | 30 | 90 | 1.6 | 0.4 | 6.8 | 5.9 |
| 47.5 | 50 | 70 | 6.4 | 1.6 | 15.3 | 10.5 |
| 55.0* | 10 | 90 | 4.3 | - | - | - |
| 55.0 | 30 | 70 | 6.0 | 1.8 | 13.7 | 13.1 |
| 55.0 | 50 | 50 | 6.1 | 2.3 | 12.8 | 18.7 |
| 55.0 | 90 | 10 | 9.3 | 2.4 | 29.4 | 8.2 |
| 40.0 | 10 | 90 | 1.0 | 0.3 | 3.4 | 8.8 |
| 40.0 | 30 | 90 | 4.2 | 1.3 | 10.1 | 12.9 |

| | | | | | | |
|---|---|---|---|---|---|---|
| * - Data not saved, d₁₀ and d₉₀ values not recorded | | | | | | |

**Table 3:**

| **Salmeterol Xinafoate Assessment** | | | | |
|---|---|---|---|---|
| Salmeterol xinafoate concentration in methanol | d₅₀ (µm) | d₁₀ (µm) | d₉₀ (µm) | UI |
| 40gl⁻¹ | 5.1 | 1.0 | 10.9 | 9.2 |
| 60gl⁻¹ | 4.1 | 0.6 | 8.2 | 7.3 |

**Table 4**

| Solution flow rate (I/h) | Antisolvent flow rate (I/h) | Solution concentration (g/l) | D₅₀ (microns) |
|---|---|---|---|
| 30 | 70 | 45 | 2.1 |
| 30 | 70 | 45 | 2.4 |
| 30 | 70 | 50 | 2.5 |
| 50 | 90 | 40 | 4.7 |
| 10 | 70 | 45 | 2.0 |
| 30 | 50 | 45 | 3.9 |
| 10 | 50 | 40 | 1.9 |
| 30 | 70 | 45 | 2.0 |
| 50 | 70 | 45 | 5.8 |
| 30 | 70 | 40 | 3.0 |
| 10 | 90 | 50 | 4.1 |
| 50 | 50 | 50 | 4.5 |
| 30 | 90 | 45 | 3.0 |
| 40 | 70 | 45 | 4.3 |

**Table 5:**

| **Particle size of sieved and unsieved drug fractions** | | | |
|---|---|---|---|
| Screen aperture size (microns) | Particle size (microns) | | |
| | D10 | D50 | D90 |
| Unsieved | 0.7 | 2.0 | 6.6 |
| 125 | 0.7 | 2.0 | 6.9 |
| 180 | 0.7 | 2.0 | 7.2 |
| 355 | 0.7 | 2.1 | 7.0 |

## Claims

1. A process for preparing crystalline particles of a substance suitable for inhalation therapy which comprises (i) admitting a stream of solution of the substance in a liquid solvent and a stream of liquid antisolvent for said substance which is miscible with the liquid solvent tangentially into a cylindrical mixing chamber having an axial outlet port such that said streams are thereby intimately mixed through formation of a vortex and precipitation of crystalline particles of the substance is thereby caused; and (ii) collecting the resultant crystalline particles suspended in the stream of liquid discharged from the outlet port of the mixing chamber.

2. A process as claimed in claim 1 wherein, prior to collecting the resultant crystalline particles, the stream of liquid discharged from the outlet port of the mixing chamber is diluted with liquid anti-solvent.

3. An apparatus for preparing crystalline particles of a substance suitable for inhalation therapy which comprises
(i) a first reservoir (3) of said substance dissolved in a liquid solvent;
(ii) a second reservoir (3) of liquid antisolvent for said substance which is miscible with the liquid solvent;
(iii) a cylindrical mixing chamber (2) having first and second tangential inlet ports (11) and an axial outlet port (14);
(iv) means for delivering the contents of the first and second reservoirs to the mixing chamber in a stream via the first and second inlet ports respectively at independent controlled flow rate wherein the streams of liquid from the inlet and outlet ports are intimately mixed in the cylindrical mixing chamber through formation of a vortex and precipitation of crystalline particles of the substance is thereby caused; and
(vi) means (5) for collecting crystalline particles suspended in the stream of liquid discharged from the outlet port of the mixing chamber.

4. An apparatus as claimed in claim 3 further comprising means (7) for diluting the stream of liquid discharged from the outlet port of the mixing chamber by delivering a stream of anti-solvent to said stream of liquid downstream of the outlet port of the mixing chamber and upstream of the means for collecting the crystalline particles.

5. An apparatus according to claim 3 or claim 4 wherein the means for delivering the contents of the first and second reservoirs to the mixing chamber via the first and second inlet ports respectively at independent controlled flow rate comprises one or more pumps (4).

6. An apparatus according to any one of claims 3 to 5 wherein the means for collecting crystalline particles suspended in the stream of liquid discharged from the outlet port of the mixing chamber comprises a filter.

7. An apparatus according any one of claims 3 to 5 wherein the means for collecting crystalline particles suspended in the stream of liquid discharged from the outlet port of the mixing chamber comprises a hydrocyclone or an ultrafiltration facility.

8. An apparatus according to any one of claims 3 to 7 wherein the means for collecting crystalline particles suspended in the stream of liquid discharged from the outlet port of the mixing chamber comprises a spray drying facility.

9. A process according to claim 1 or claim 2 using an apparatus according to any one of claims 3 to 8 which comprises:
(i) delivering the contents of the first and second reservoirs to the mixing chamber via the first and second inlet ports respectively at independent controlled flow rate; and
(iii) collecting the crystalline particles suspended in the stream of liquid discharged from the outlet port of the mixing chamber.

10. A process according to claim 9 wherein the substance is a pharmaceutical substance suitable for inhalation therapy.

11. A process according to claim 9 wherein the substance is a carrier substance suitable for inhalation therapy.

12. A process according to claim 10 wherein the substance is fluticasone, beclomethasone, salmeterol, salbutamol or an ester, salt or solvate thereof.

13. A process according to claim 11 wherein the substance is lactose.

14. A process according to claim 12 wherein the substance is fluticasone propionate.

15. A process according to claim 12 wherein the substance is salmeterol xinafoate.

16. A process according to claim 14 wherein the solvent is acetone and the anti-solvent is water.

17. A process according to claim 15 wherein the solvent is methanol and the anti-solvent is water

18. A population of particles obtainable by a process according to any one of claims 1, 2 and 9 to 17.

19. A pharmaceutical composition comprising a population of particles according to claim 18.

20. A pharmaceutical aerosol composition comprising a population of particles obtainable by a process according to claim 10 and a liquefied hydrofluoroalkane as propellant.

21. A pharmaceutical dry powder composition comprising a population of particles obtainable by a process according to claim 10 and lactose as carrier.
